Europäisches Patentamt

European Patent Office

Office européen des brevets

(11) Numéro de publication : **0 379 396 B1**

(12)

# FASCICULE DE BREVET EUROPEEN

(45) Date de publication du fascicule du brevet :
**26.08.92 Bulletin 92/35**

(51) Int. Cl.⁵ : **C07C 22/00,** C07C 17/00,
B01J 23/46

(21) Numéro de dépôt : **90400048.6**

(22) Date de dépôt : **08.01.90**

(54) **Hydrogénolyse sélective de dérivés Perhalogènes de l'éthane.**

(30) Priorité : **19.01.89 FR 8900601**

(43) Date de publication de la demande :
**25.07.90 Bulletin 90/30**

(45) Mention de la délivrance du brevet :
**26.08.92 Bulletin 92/35**

(84) Etats contractants désignés :
**AT BE CH DE DK ES FR GB GR IT LI LU NL SE**

(56) Documents cités :
**EP-A- 0 164 954
FR-A- 2 116 524
US-A- 3 505 417**

(73) Titulaire : **ELF ATOCHEM S.A.
4 & 8, Cours Michelet La Défense 10
F-92800 Puteaux (FR)**

(72) Inventeur : **Cheminal, Bernard
La Rivière, Orliénas
F-69530 Brignais (FR)**
Inventeur : **Cognion, Jean-Marie
85 Route de Charly
F-69230 Saint-Genis Laval (FR)**
Inventeur : **Guillet, Dominique
2 Route de Vourlses - Bt B
F-69230 Saint-Genis Laval (FR)**

(74) Mandataire : **Leboulenger, Jean et al
ATOCHEM Département Propriété Industrielle
F-92091 Paris la Défense 10 Cédex 42 (FR)**

## Description

La présente invention concerne la fabrication des chlorofluoroéthanes de formule :

$$CF_3\text{-}CHF_xCl_{2\text{-}x} \quad \text{(I)}$$

où x est égal à 0 ou 1, par hydrogénation catalytique d'un perhalogéno-éthane de formule :

$$CF_3\text{-}CF_xCl_{3\text{-}x} \quad \text{(II)}$$

Les deux matières premières, incluses dans la formule (II), sont le trichloro-1,1,1 trifluoro-2,2,2 éthane ($CF_3CCl_3$) et le dichloro-1,1 tétrafluoro-1,2,2,2 éthane ($CF_3CFCl_2$) dont la substitution d'un atome de chlore par un atome d'hydrogène conduit respectivement au dichloro-1,1 trifluoro-2,2,2 éthane ($CF_3CHCl_2$) et au chloro-1 tétrafluoro-1,2,2,2 éthane ($CF_3CHFCl$).

L'hydrogénation catalytique des composés (II) à déjà été décrite, mais les sélectivités en produit correspondant à l'élimination d'un seul atome de chlore sont faibles. Ainsi, l'hydrogénolyse du dichloro-1,1 tétrafluoro-1,2,2,2 éthane à 280°C sur un catalyseur à 5 % de palladium sur charbon (brevet GB 1 578 933) fournit un produit contenant 70 % de tétrafluoro-1,1,1,2 éthane. Des résultats semblables sont obtenus par C. GERVASUTTI et al (Journal of Fluorine Chemistry 1981, 1, 1-20) sur un catalyseur à 0,5 % de palladium sur charbon : à 170°C, l'hydrogénolyse du dichloro-1,1 tétrafluoro-1,2,2,2 éthane conduit à 76 % de tétrafluoro-1,1,1,2 éthane. Pour résoudre le problème de l'élimination d'un seul atome de chlore, il faut recourir selon la demande de brevet japonais 106051/82 (publication JP 222038/83) à une réduction chimique par le couple zinc-éthanol ; dans les conditions décrites, la sélectivité de l'hydrogénolyse du trichloro-1,1,1 trifluoro-2,2,2 éthane en dichloro-1,1 trifluoro-2,2,2 éthane atteint 90 %. Cependant, ce procédé présente l'inconvénient d'employer du zinc métallique coûteux et de sous-produire du chlorure de zinc qu'il faut détruire.

Il a maintenant été trouvé que l'élimination catalytique d'un seul atome de chlore s'effectue très sélectivement si l'on utilise un catalyseur à base de ruthénium.

La présente invention a donc pour objet un procédé de préparation des chlorofluoroéthanes de formule (I) par hydrogénation catalytique d'un perhalogéno-éthane de formule (II), caractérisé en ce que l'on emploie un catalyseur à base de ruthénium déposé sur un support.

Dans la catalyseur utilisé selon l'invention, la teneur en ruthénium peut aller de 0,1 à 10 % en poids, mais est de préférence comprise entre 0,2 et 8 %.

Le support peut être de nature très diverse et choisi, par exemple, parmi les alumines, le fluorure d'aluminium et les charbons actifs. On préfère comme supports les charbons ayant une surface spécifique comprise entre 200 et 1500 m²/g (de préférence entre 600 et 1200 m²/g), une porosité élevée (0,3 à 0,7 cm³/g) et une granulométrie compatible avec une catalyse en lit fixe (1 à 10 mm). Ces produits sont commercialisés sous forme d'extrudés ou de billes par de nombreuses Sociétés.

Le catalyseur selon l'invention peut être préparé par imprégnation du support avec une solution aqueuse ou organique d'un dérivé du ruthénium, évaporation de l'eau ou du solvant et traitement thermique à une température allant de 200 à 600°C (de préférence 300 à 450°C) et sous courant d'hydrogène (de préférence sous une pression de 1 à 5 bars) pour libérer le ruthénium. La nature du dérivé de ruthénium utilisé est sans importance et peut être, par exemple, un chlorure, un nitrate, un acide chlororuthénique, un sel d'ammonium ou un acétylacétonate.

Le catalyseur selon l'invention peut également être choisi parmi les produits disponibles sur le marché, par exemple ceux de la Société ENGELHARD qui propose des catalyseurs contenant de 0,5 à 5 % de ruthénium sur des alumines ou des charbons extrudés.

L'hydrogénation catalytique selon l'invention peut être effectuée à une température allant de 50 à 250°C avec un rapport molaire hydrogène/perhalogénoéthane (II) allant de 0,5 à 4, sous une pression de 1 à 20 bars (de préférence 1 à 5 bars) et un débit horaire de 1 à 20 moles de perhalogénoéthane (II) par litre de catalyseur.

Les exemples suivants illustrent l'invention, sans la limiter. Dans les exemples 2 à 4, les résultats sont exprimés par le taux de transformation globale ($TT_G$) et la sélectivité (S) en un produit de la réaction:

$$TT_G = 100 \times \frac{\text{Nombres de moles de composés (II) transformé}}{\text{Nombre de moles de composé (II) introduit}}$$

$$S = 100 \times \frac{\text{Nombre de moles de produit formé}}{\text{Nombre de moles de composé (II) transformé}}$$

les analyses à l'entrée et en sortie du réacteur étant effectuées par chromatographie phase vapeur en ligne.

## EXEMPLE 1 : Préparation des catalyseurs

Dans un évaporateur rotatif, on charge 50 ml (23 g) d'un charbon actif ayant une porosité de 0,6 cm³/g et une surface spécifique de 950 m²/g sous forme d'extrudés de 1,8 mm de diamètre. Après dégazage pendant 3 heures à 100°C sous pression réduite (1 kPa), on introduit 70 ml d'une solution aqueuse de trichlorure de

ruthénium hydraté RuCl$_3$, xH$_2$O contenant 1,5 g de ruthénium, puis on évapore l'eau sous pression réduite (1 kPa) et sèche à 100°C. On traite ensuite à 400°C pendant 2 heures sous un courant d'hydrogène (10 N1/h) et obtient ainsi un catalyseur contenant 6 % de ruthénium (catalyseur A).

En opérant de la même façon, mais avec une solution aqueuse contenant 0,12 g de ruthénium, on obtient un catalyseur à 0,5 % de ruthénium (catalyseur B).

## EXEMPLE 2

Dans une tube en Inconel de 45 cm de long et de 2,72 cm de diamètre intérieur, chauffé électriquement, on introduit 50 ml du catalyseur A décrit à l'exemple 1, puis on y fait passer un mélange d'hydrogène et de dichloro-1,1 tétrafluoro-1,2,2,2 éthane aux rapports molaires, débits et températures indiqués dans le Tableau suivant dont la dernière partie rassemble les résultats obtenus.

### TABLEAU 1

| ESSAI N° | 1 | 2 | 3 | 4 | 5 |
|---|---|---|---|---|---|
| Conditions opératoires : | | | | | |
| Température (°C) | 150 | 200 | 200 | 200 | 200 |
| Rapport molaire H$_2$/C$_2$F$_4$Cl$_2$ | 4 | 4 | 1 | 1 | 0,5 |
| Débit C$_2$F$_4$Cl$_2$ (mole/heure) | 0,07 | 0,07 | 0,18 | 0,08 | 0,10 |
| Résultats | | | | | |
| % TT$_G$ du C$_2$F$_4$Cl$_2$ | 43 | 91 | 40 | 56 | 33 |
| % S en CF$_3$CHFCl | 49 | 82 | 84 | 87 | 88 |
| % S en CF$_3$CH$_3$ | 50 | 16 | 14 | 11 | 11 |

Dans la plupart des cas, la sélectivité de l'hydrogénolyse d'une seule liaison C-Cl est supérieure à 80 %.

A titre comparatif, on a reproduit les essais n° 1 et 2, mais en remplaçant le catalyseur A selon l'invention par un catalyseur à 5 % de palladium préparé de la même façon et sur le même support qu'à l'exemple 1 avec PdCl$_2$ au lieu de RuCl$_3$. Les résultats, rassemblés dans le Tableau 2 suivant, montrent qu'avec ce catalyseur au palladium, la sélectivité de la réaction est nettement en faveur de l'arrachement de deux atomes de chlore.

## TABLEAU 2

| ESSAI COMPARATIF N° | 6 | 7 |
|---|---|---|
| **Conditions opératoires :** | | |
| Température (°C) | 150 | 200 |
| Rapport molaire $H_2/C_2F_4Cl_2$ | 4 | 4 |
| Débit $C_2F_4Cl_2$ (mole/heure) | 0,07 | 0,07 |
| **Résultats :** | | |
| % $TT_G$ du $C_2F_4Cl_2$ | 100 | 100 |
| % S en $CF_3CHFCl$ | 4 | 3 |
| % S en $CF_3CH_3$ | 1 | 1,2 |
| % S en $CF_3CH_2F$ | 94,5 | 95 |

## EXEMPLE 3

Dans le même appareillage qu'à l'exemple 2, on introduit 50 ml d'une nouvelle charge de catalyseur A sur laquelle on effectue successivement différents essais d'hydrogénation du trichloro-1,1,1 trifluoro-2,2,2 éthane ($CF_3$-$CCl_3$).

Les conditions opératoires des essais et les résultats obtenus sont rassemblés dans le Tableau 3 suivant. A côté du produit attendu, le dichloro-1,1 trifluoro-2,2,2 éthane ($CF_3CHCl_2$), on trouve principalement comme sous-produit le trifluoro-1,1,1 éthane ($CF_3CH_3$) et, dans certains cas, des produits oléfiniques de condensation en $C_4$.

4

TABLEAU 3

| ESSAI N° | CONDITIONS OPERATOIRES | | | | RESULTATS | | |
|---|---|---|---|---|---|---|---|
| | Temp. °C | Rapport molaire $H_2/C_2F_3Cl_3$ | Débit $C_2F_3Cl_3$ (moles/h) | $\% \, TTG$ du $C_2F_3Cl_3$ | $\% \, S$ en $CF_3CHCl_2$ | $\% \, S$ en $CF_3CH_3$ |
| 11 | 50 | 0,83 | 0,12 | 10 | 100 | – |
| 12 | 100 | 0,83 | 0,12 | 28,5 | 94 | 4 |
| 13 | 110 | 0,83 | 0,12 | 42,5 | 89 | 3 |
| 14 | 150 | 1 | 0,10 | 70 | 77 | 3 |
| 15 | 170 | 0,90 | 0,14 | 74 | 79 | 4,5 |
| 16 | 200 | 0,83 | 0,12 | 68 | 59 | 3,5 |
| 17 | 115 | 0,56 | 0,19 | 59 | 63 | 1,6 |
| 18 | 100 | 0,91 | 0,14 | 64 | 79 | 2,1 |
| 19 | 100 | 1,6 | 0,096 | 80 | 80 | 3 |
| 20 | 100 | 3 | 0,093 | 55 | 80 | 4,2 |
| 21 | 150 | 3 | 0,093 | 87 | 53 | 8,3 |
| 22 | 100 | 3 | 0,058 | 59 | 92 | 6,3 |

**EXEMPLE 4**

Dans le même appareillage qu'à l'exemple 2 et avec une charge de 50 ml de catalyseur, on a procédé à différents essais d'hydrogénolyse du trichloro-1,1,1 trifluoro-2,2,2 éthane en utilisant les catalyseurs B, C et D suivants :

– B : catalyseur à 0,5 % de Ru sur charbon décrit au dernier paragraphe de l'exemple 1,

– C : catalyseur à 1 % de palladium sur charbon, préparé comme à l'exemple 1, mais avec $PdCl_2$ au lieu de $RuCl_3$,

– D : catalyseur à 5 % de platine sur charbon, préparé comme à l'exemple 1, mais avec $PtCl_6H_2$ au lieu de $RuCl_3$.

Les conditions opératoires et les résultats de ces essais sont rassemblés dans le Tableau 4 suivant :

TABLEAU 4

| ESSAI N° | Selon l'invention | | Comparatifs | | | |
|---|---|---|---|---|---|---|
| | 31 | 32 | 33 | 34 | 35 | 36 |
| **Conditions opératoires** | | | | | | |
| Catalyseur | B | B | C | D | D | D |
| Température (°C) | 200 | 150 | 150 | 80 | 100 | 125 |
| Rapport molaire $H_2/C_2F_3Cl_3$ | 0,5 | 2,5 | 0,5 | 1,4 | 1,4 | 1,8 |
| Débit $C_2F_3Cl_3$ (mole/h) | 0,12 | 0,6 | 0,11 | 0,10 | 0,10 | 0,10 |
| **Résultats** | | | | | | |
| % $TT_G$ du $C_2F_3Cl_3$ | 21 | 6 | 18 | 92 | 93,5 | 100 |
| % S en $CF_3CHCl_2$ | 80 | 100 | 28 | 64 | 64 | 27 |
| % S en $CF_3CH_3$ | 0 | 0 | 72 | 32 | 34 | 48 * |

(*) S en $CF_3CH_2Cl$ = 20 %

## Revendications

1. Procédé de préparation de chlorofluoroéthanes de formule :
$$CF_3\text{-}CHF_xCl_{2\text{-}x} \quad (I)$$
où x est égal à 0 où 1, par hydrogénation catalytique d'un perhalogéno-éthane de formule :
$$CF_3\text{-}CF_xCl_{3\text{-}x} \quad (II)$$
caractérisé en ce qu'on utilise un catalyseur à base de ruthénium déposé sur un support.

2. Procédé selon la revendication 1, caractérisé en ce que le catalyseur a une teneur en ruthénium allant de 0,1 à 10 % en poids, de préférence entre 0,2 et 8 %.

3. Procédé selon la revendication 1 ou 2, caractérisé en ce que le support est une alumine, le fluorure, d'aluminium ou un charbon actif.

4. Procédé selon la revendication 1 ou 2, caractérisé en ce que le support est un charbon actif ayant une surface spécifique comprise entre 200 et 1500 m²/g, une porosité de 0,3 à 0,7 cm³/g et une granulométrie de 1 à 10 mm.

5. Procédé selon l'une des revendications 1 à 4, dans lequel l'hydrogénation est effectuée à une température

6

comprise entre 50 et 250°C et sous une pression de 1 à 20 bars, de préférence 1 à 5 bars.

6. Procédé selon l'une des revendications 1 à 5, dans lequel le rapport molaire hydrogène/perhalogénoéthane (II) est compris entre 0,5 et 4.

7. Procédé selon l'une des revendications 1 à 6, dans lequel le débit horaire de perhalogéno-éthane (II) est de 1 à 20 moles par litre de catalyseur.

## Patentansprüche

1. Verfahren zur Herstellung von Chlorfluorethanen der Formel
$$CF_3\text{-}CHF_xCl_{2-x} \quad (I)$$
worin x gleich 0 oder 1 ist, durch katalytische Hydrierung einer Perhalogenethanverbindung der Formel
$$CF_3\text{-}CF_xCl_{3-x} \quad (II)$$
dadurch gekennzeichnet, daß man einen auf einem Substrat abgelagerten Rutheniumkatalysator verwendet.

2. Verfahren gemäß Anspruch 1, dadurch gekennzeichnet, daß der Katalysator einen Rutheniumgehalt von 0,1 bis 10 Gew.%, vorzugsweise 0,2 bis 8 % aufweist.

3. Verfahren gemäß Anspruch 1 oder 2, dadurch gekennzeichnet, daß das Substrat Tonerde, Aluminiumfluorid oder Aktivkohle ist.

4. Verfahren gemäß Anspruch 1 oder 2, dadurch gekennzeichnet, daß das Substrat Aktivkohle mit einer spezifischen Oberfläche zwischen 200 und 1500 $m^2$/g, einer Porosität von 0,3 bis 0,7 $cm^3$/g und einer Korngröße von 1 bis 10 mm ist.

5. Verfahren gemäß einem der Ansprüche 1 bis 4, dadurch gekennzeichnet, daß die Hydrierung bei einer Temperatur zwischen 50 und 250°C und unter einem Druck von 1 bis 20 bar, vorzugsweise 1 bis 5 bar durchgeführt wird.

6. Verfahren gemäß einem der Ansprüche 1 bis 5, dadurch gekennzeichnet, daß das Molverhältnis Wasserstoff/Perhalogenethan (II) zwischen 0,5 und 4 liegt.

7. Verfahren gemäß einem der Ansprüche 1 bis 6, dadurch gekennzeichnet, daß der stündliche Durchsatz von Perhalogenethan (II) 1 bis 20 Mole pro Liter Katalysator beträgt.

## Claims

1. Process for preparing chlorofluoroethanes of the formula:
$$F_3\text{-}CHF_xCl_{2-x} \quad (I)$$
where x is equal to 0 or 1, by the catalytic hydrogenation of a perhaloethane of the formula:
$$F_3\text{-}CF_xCL_{3-x} \quad (II)$$
characterised in that a ruthenium-based catalyst deposited on a support is used.

2. Process according to Claim 1, characterised in that the catalyst has a ruthenium content ranging from 0.1 to 10% by weight, and preferably between 0.2 and 8%.

3. Process according to Claim 1 or 2, characterised in that the support is an alumina, aluminium fluoride or an active charcoal.

4. Process according to Claim 11 or 2, characterised in that the support is an active charcoal having a specific surface area of between 200 and 1500 $m^2$/g, a porosity of 0.3 to 0.7 $cm^3$/g and a particle size of 1 to 10 mm.

5. Process according to one of Claims 1 to 4, in which the hydrogenation is performed at a temperature of between 50 and 250°C and under a pressure of 1 to 20 bars, and preferably 1 to 5 bars.

6. Process according to one of Claims 1 to 5, in which the mole ratio hydrogen/perhaloethane (II) is between 0.5 and 4.

7. Process according to one of Claims 1 to 6, in which the hourly flow rate of perhaloethane (II) is from 1 to 20 moles per litre of catalyst.